# EUROPÄISCHE PATENTSCHRIFT

(11) **EP 2 870 949 B1**
(45) Veröffentlichungstag und Bekanntmachung des Hinweises auf die Patenterteilung: **25.05.2016**
(21) Anmeldenummer: 13192449.0
(22) Anmeldetag: 12.11.2013
(51) Int. Cl.: A61F 11/00

(54) **Mechanischer Tinnitus-Noiser**
Mechanical tinnitus noiser
Bruiteur d'acouphènes mécanique

(43) Veröffentlichungstag der Anmeldung: 13.05.2015
(73) Patentinhaber: Draxler-Schinko, Julia, 4125 Riehen (CH)
(72) Erfinder: Draxler-Schinko, Julia, 4125 Riehen (CH)
(74) Vertreter: Wirnsberger, Gernot

(56) Entgegenhaltungen:
- WO-A1-99/07302
- WO-A2-02/30157
- CH-A- 74 488
- US-A1- 2013 152 949

## Beschreibung

Die Erfindung betrifft eine in der Ohrmuschel tragbare Vorrichtung zum Überdecken eines Tinnitusgeräusches, wobei zumindest zwei Körper vorgesehen sind, welche relativ zueinander beweglich gelagert sind, um durch Aneinanderstoßen ein das Tinnitusgeräusch überdeckendes Geräusch zu erzeugen.

Weiter betrifft die Erfindung ein Verfahren zum Herstellen einer Vorrichtung zum Überdecken eines Tinnitusgeräusches, insbesondere durch eine in der Ohrmuschel tragbare Vorrichtung.

Grundsätzlich muss bei Tinnitus zwischen objektivem und subjektivem Tinnitus unterschieden werden. Der selten auftretende objektive Tinnitus ist für einen behandelnden Arzt hör- und messbar und kann meist medikamentös behandelt werden. Subjektiver Tinnitus ist eine akustische Wahrnehmung, welche von einem Betroffenen bzw. Patienten hörbar ist, ohne dass Schall von außen auf das Ohr wirkt. Als Ursache für einen Tinnitus kommen mehrere Faktoren infrage. Aller Wahrscheinlichkeit nach handelt es sich dabei um einen Phantomschmerz, welcher in vielen Fällen mit einem Hörsturz einhergeht. Tinnitus ist also nur ein Symptom und keine Krankheit. Da das Tinnitusgeräusch nur vom betroffenen Patienten selbst und nicht von anderen Menschen hörbar ist, ist dieses entsprechend schwer zu behandeln. Weiter kann Tinnitus nur symptomatisch behandelt werden, da die eigentliche Ursache nur in den wenigsten Fällen klar feststellbar ist. Folglich wird in erster Linie versucht, die Ohrgeräusche eines Patienten zu lindern.

Aus dem Stand der Technik sind Vorrichtungen zur Behandlung von Tinnitus bekannt. Dabei werden prinzipiell zwei Arten von Vorrichtungen unterschieden. Zum einen gibt es Vorrichtungen, welche nach dem Prinzip der Tinnitus-Maskierung arbeiten. Bei diesen Vorrichtungen, den sogenannten Tinnitus-Maskern, wird die Frequenz des Tinnitus eines Patienten ermittelt und in weiterer Folge das Ohr des Patienten mit einer entsprechenden Frequenz akustisch stimuliert. Bei der dabei verwendeten Frequenz handelt es sich meist um eine der Tinnitusfrequenz des Patienten gegenläufige Frequenz, welche für jeden Patienten individuell ermittelt wird.

Zum anderen sind Vorrichtungen zur Behandlung eines Tinnitus bekannt, durch welche ein Geräusch zum Ohr eines Patienten führbar ist, welches größer ist als das Tinnitusgeräusch selbst. Solche Vorrichtungen werden Tinnitus-Noiser genannt und arbeiten nach dem Prinzip des Überdeckens eines Tinnitusgeräusches. Dieses Beschallen des Ohres eines Patienten lenkt diesen von seinem Ohrgeräusch ab.

All diesen Vorrichtungen ist gemein, dass diese zumindest teilweise elektrisch bzw. elektronisch arbeiten. Die Geräusche bzw. Töne, mit welchen das Ohr eines Patienten beschallt wird, werden dabei elektrisch und mithilfe elektrischer bzw. elektronischer Bauteile erzeugt. Erfahrungsgemäß sind elektrische bzw. elektronische Bauteile, welche aufgrund der Größe einer in der Ohrmuschel tragbaren Vorrichtung sehr klein gehalten werden müssen, relativ teuer. Des Weiteren haben Vorrichtungen dieser Art den Nachteil, dass diese leicht fehleranfällig und, wenn überhaupt, nur umständlich auf individuelle Bedürfnisse anpassbar sind. Individuelle Bedürfnisse eines Tinnituspatienten können sich mehrmals am Tag ändern, da diese unter anderem von Umgebungsgeräuschen und persönlichem Wohlergehen abhängig sind. Demnach ist das Tinnitusgeräusch nicht immer gleich stark wahrnehmbar und muss nicht immer mit Geräuschen der gleichen Lautstärke überdeckt werden.

Darüber hinaus offenbaren die CH 74 488 A und die US 2013/0152949 A1 jeweils eine Vorrichtung zum Überdecken eines Explosionsgeräusches, wobei diese in einen Gehörgang einführbar sind und zwei relativ zueinander bewegliche Körper umfassen.

Vorrichtungen zum Überdecken eines Tinnitusgeräusches werden üblicherweise in mehreren Schritten hergestellt. Bei einer individuellen Anfertigung wird in einem ersten Schritt ein Abdruck einer Ohrmuschel eines Patienten angefertigt, wonach eine Form als Negativ dieses Abdruckes hergestellt wird. Anschließend wird die Vorrichtung in diese Form gegossen. Nachteilig bei diesem Verfahren sind das aufwendige Herstellen von Formen sowie das Formenwechseln beim Herstellen einer anderen Vorrichtung, auch wenn diese sich nur minimal von einer ersten Vorrichtung unterscheidet. Des Weiteren muss eine so produzierte Vorrichtung zur Vollendung des Herstellungsprozesses noch von Hand nachbearbeitet werden, um kleine Unebenheiten bzw. Fehler zu beseitigen. Es ist auch möglich, eine Vorrichtung zum Überdecken eines Tinnitusgeräusches anhand eines virtuellen Modells zu erzeugen. Diese Technik ist allgemein unter CAD bekannt. Ein Verfahren zur Herstellung von Hörhilfen ist beispielsweise in der WO 02/30157 A2 offenbart.

Weiter sind Vorrichtungen zum Überdecken eines Tinnitusgeräusches bekannt, welche nicht individuell angepasst, sondern Standardvorrichtungen sind, welche für ein durchschnittliches Ohr hergestellt werden. Diese Art von Vorrichtungen hat den Nachteil, dass diese für einen Patienten nicht besonders angenehm zu tragen ist bzw. als störend empfunden wird, insbesondere bei einer längeren Tragezeit.

Aufgabe der Erfindung ist es, eine Vorrichtung der eingangs genannten Art anzugeben, welche in einfacher Weise individuell und von einem Patienten selbst steuerbar ist.

Eine weitere Aufgabe der Erfindung ist es, ein Verfahren der eingangs genannten Art anzugeben, mit welchem eine Vorrichtung zum Überdecken eines Tinnitusgeräusches, welche in einfacher Weise individuell und von einem Patienten selbst steuerbar ist, relativ einfach und in wenigen Arbeitsschritten hergestellt werden kann.

Die erste Aufgabe wird erfindungsgemäß dadurch gelöst, dass bei einer Vorrichtung der eingangs genannten Art die zumindest zwei Körper als kugelförmige Körper ausgebildet sind.

Ein mit der Erfindung erzielter Vorteil ist insbesondere darin zu sehen, dass nur wenige Bauteile benötigt werden, um ein Geräusch zu erzeugen, welches das Tinnitusgeräusch überdeckt. Weiter ist durch die zueinander beweglich gelagerten Körper das tinnitusüberdeckende Geräusch in einfacher Art und Weise erzeugbar, nämlich durch Kopfbewegungen bzw. Körperbewegungen des Patienten, der die Vorrichtung trägt. Bei Kopfbewegungen werden die zueinander beweglich gelagerten Körper in Bewegung versetzt und dadurch ein Geräusch erzeugt. Ein Patient, der die Vorrichtung trägt, kann in einfacher Weise durch Ändern seiner Kopf- bzw. Körperbewegungen die Bewegungen der beweglich zueinander gelagerten Körper beeinflussen. Damit ist in weiterer Folge die Lautstärke des durch Aneinanderstoßen erzeugten Geräusches veränderbar. So kann ein Patient die Vorrichtung selbst steuern bzw. die Lautstärke der Vorrichtung selbst und in einfacher Weise auf sich verändernde Umstände anpassen. Somit eignet sich die erfindungsgemäße Vorrichtung vorzüglich zur Behandlung von subjektivem Tinnitus.

Vorteilhaft ist es, wenn an einem ersten Ende, welches in den Gehörgang einführbar ist, zumindest eine Öffnung ausgebildet ist. Tinnituspatienten berichten darüber, dass das hörbare Geräusch lauter wird, wenn der Gehörgang verschlossen ist. Aus diesem Grund ist es wichtig, dass dieser offen bleibt, um ein wirksames Überdecken des Tinnitusgeräusches zu gewährleisten. Darüber hinaus ist durch das in den Gehörgang einführbare offene Ende sichergestellt, dass das durch Aneinanderstoßen erzeugte Geräusch in unmittelbarer Nähe des Trommelfells des Patienten erzeugt wird und somit für diesen gut hörbar ist.

Es ist von Vorteil, wenn ein zweites Ende, welches die Vorrichtung nach außen hin abschließt, zumindest eine Öffnung aufweist. Dadurch findet stets ein Luftaustausch zwischen Ohr und Umgebung statt. Weiter ist durch die Öffnung ein Wahrnehmen normaler Umgebungsgeräusche weiterhin möglich. Die Öffnungen am ersten Ende und am zweiten Ende können miteinander durch einen gasdurchlässigen Kanal verbunden sein.

Zweckmäßig ist es, dass zur Erzeugung des Geräusches zumindest ein erster Körper frei beweglich in einem zweiten Körper gelagert ist, wobei der zweite Körper als Hohlkörper ausgebildet ist. Dabei ist der erste Körper in Bewegung versetzbar und erzeugt beim Zusammenstoß mit dem zweiten Körper ein Geräusch, welches das Tinnitusgeräusch überdeckt. Hierbei kann vorgesehen sein, dass der zweite Körper positionsstabil an oder in der Vorrichtung fixiert ist.

Bevorzugt ist vorgesehen, dass der zumindest eine zweite Körper zumindest eine Öffnung aufweist, wobei die zumindest eine Öffnung kleiner als der zumindest eine erste Körper ist. Dadurch funktioniert der zweite Körper wie ein Klangkörper, um die erzeugten Geräusche zu verstärken.

Es ist vorgesehen, dass die zumindest zwei Körper als kugelförmige Körper ausgebildet sind. Kugelförmige Körper gewährleisten, dass das durch Aneinanderstoßen der Körper erzeugte Geräusch relativ gleichmäßig klingt. Der zumindest eine erste Körper kann dabei als volle oder als hohle Kugel ausgebildet sein. Diese Art von Kugel ist drehsymmetrisch bezüglich jeder Achse durch den Mittelpunkt. Diese Eigenschaft gewährleistet eine gleichmäßige Bewegung der Kugeln unabhängig von deren Ausgangsposition. Bei einer weiteren Variante der Vorrichtung kann ein als kugelförmig ausgebildeter Körper einen Schwerpunkt abseits vom Mittelpunkt aufweisen. Durch die Position des Schwerpunktes ist ein Bewegungsablauf eines sich bewegenden kugelförmigen Körpers steuerbar und damit auch die Geräusche, welche durch Aneinanderstoßen mehrerer Körper erzeugt werden.

Vorteilhaft kann vorgesehen sein, dass die zueinander gerichteten Oberflächen der zumindest zwei Körper als raue Oberflächen ausgebildet sind, um die Lautstärke der durch Aneinanderstoßen der Körper erzeugten Geräusche zu erhöhen.

Die zumindest zwei Körper sind relativ zueinander in Bewegung versetzbar, wobei die Bewegung frei von elektrischen Mitteln erzeugbar ist. Die Bewegung der zwei Körper relativ zueinander wird dabei durch bloßes Bewegen des Kopfes eines die Vorrichtung tragenden Patienten erzeugt. Durch diese Maßnahme ist die Lautstärke des Tinnitus überdeckenden Geräusches jederzeit vom Patienten selbst steuerbar und muss nicht umständlich über elektronische Hilfsmittel geregelt werden. Weiter kann ein Patient sofort auf sich ändernde Umstände, welche das hörbare Tinnitusgeräusch beeinflussen und verändern, reagieren.

Es hat sich bewährt, dass keine elektrischen Bauelemente vorhanden sind, um die Ausführung der Vorrichtung so einfach wie möglich zu halten.

Bevorzugt kann vorgesehen sein, dass die Vorrichtung der Ohrform passgenau anpassbar ist. Eine sogenannte Otoplastik wird der Ohrform jedes Patienten individuell angepasst, wofür eine individuelle Abformung des Ohres des Patienten die Grundlage darstellt. Eine Otoplastik ist für den Patienten überaus komfortabel zu tragen und wirkt sich nicht störend auf das alltägliche Leben aus. Erfindungsgemäße Vorrichtungen können jedoch auch so ausgebildet sein, dass diese zwar etwa als Negativ einer menschlichen Ohrmuschel angefertigt sind. In diesem Fall werden die Vorrichtungen nicht für jeden Patienten individuell angefertigt und sind somit für den größten Teil der Patienten gleichermaßen tragbar. Eine weitere Ausführungsform der Vorrichtung liegt vor, wenn diese nicht passgenau der Ohrform angepasst ist, sondern eine längliche Gestalt aufweist, welche in den Gehörgang einführbar ist.

Vorteilhaft ist, wenn die Vorrichtung mit einem 3D-Drucker hergestellt ist. Dadurch ist die Vorrichtung als Ganzes produzierbar und kann relativ einfach und schnell hergestellt werden. Weiter produziert eine Vorrichtung, welche mit einem 3D-Drucker hergestellt ist, keinen Materialabfall und es ist auch keine Herstellung von Formen oder dergleichen notwendig.

Die weitere Aufgabe wird erfindungsgemäß dadurch gelöst, dass bei einem Verfahren der eingangs genannten Art die Vorrichtung zumindest teilweise mit einem 3D-Drucker hergestellt wird und zumindest zwei Körper relativ zueinander beweglich gelagert werden, um durch Aneinanderstoßen ein das Tinnitusgeräusch überdeckendes Geräusch zu erzeugen.

Ein Vorteil des erfindungsgemäßen Verfahrens ist insbesondere darin zu sehen, dass die Vorrichtung zum Überdecken des Tinnitus in einfacher Weise hergestellt wird. Mit einem 3D-Drucker ist es möglich, zueinander beweglich gelagerte Körper in nur einem Arbeitsschritt herzustellen. Des Weiteren entsteht bei diesem Verfahren so gut wie kein Materialabfall und es sind keine zusätzlichen Elemente wie Formen notwendig.

Es ist nicht zwingend, kann jedoch vorgesehen sein, dass ein erster Körper frei beweglich in einem zweiten Körper gelagert wird, wobei der zweite Körper als Hohlkörper ausgebildet ist. Dabei ist der zweite Körper in bzw. an der Vorrichtung fixiert und der erste Körper frei beweglich in diesem gelagert, um in einfacher Weise eine relativ gleichmäßige Bewegung zu erzeugen.

Es kann bevorzugt vorgesehen sein, dass die Vorrichtung einteilig hergestellt wird. Das einteilige Herstellen funktioniert in einfacher Weise mit einem 3D-Drucker. Im Besonderen bei der Ausführung, bei welcher ein erster Körper frei beweglich in einem zweiten Körper gelagert wird, ist diese Methode besonders gut geeignet, weil die mit beweglichen Teilen ausgebildete Vorrichtung einteilig hergestellt wird und weitgehend ohne Materialverlust sowie in einem einzigen Arbeitsschritt. Mit einem 3D-Drucker ist es möglich, die zwei zueinander beweglich gelagerten Körper der Vorrichtung in einem einzigen Arbeitsschritt herzustellen. Insbesondere wenn der zumindest eine zweite Körper zumindest eine Öffnung aufweist, wobei diese Öffnung kleiner als der zumindest eine erste Körper ist, ist es in einfacher Weise möglich, die Vorrichtung in einem einzigen Arbeitsschritt herzustellen, da nicht gedrucktes Material durch die Öffnung abrinnen kann. Es kann auch vorgesehen sein, dass die Vorrichtung aus mehreren Teilen hergestellt wird, wobei zumindest ein Teil mit einem 3D-Drucker hergestellt wird und die Teile insbesondere durch Kleben zusammengeführt werden.

Von Vorteil ist es auch, dass ein Abdruck einer menschlichen Ohrmuschel angefertigt wird, wonach ein digitales Modell der Ohrmuschel erzeugt wird und in weiterer Folge die Vorrichtung mit einem 3D-Drucker hergestellt wird. Damit wird eine Vorrichtung zum Überdecken eines Tinnitusgeräusches für jeden Patienten individuell in wenigen Arbeitsschritten hergestellt.

Weitere Merkmale, Vorteile und Wirkungen der Erfindung ergeben sich aus den dargestellten Ausführungsbeispielen. In den Zeichnungen, auf welche dabei Bezug genommen wird, zeigen:
Fig. 1 eine Vorrichtung gemäß dem Stand der Technik;
Fig. 2 eine weitere Variante der erfindungsgemäßen Vorrichtung;
Fig. 3 die Vorrichtung gemäß Fig. 2 in einem Querschnitt entlang der Linie III-III in Fig. 2;
Fig. 4 eine weitere Variante der Vorrichtung gemäß Fig. 2 in einem Querschnitt entlang analog der Linie III-III in Fig. 2;
Fig. 5 eine weitere Variante der erfindungsgemäßen Vorrichtung;
Fig. 6 die Vorrichtung gemäß Fig. 5 in einem Querschnitt entlang der Linie VI-VI in Fig. 5;
Fig. 7 eine weitere Variante der erfindungsgemäßen Vorrichtung;
Fig. 8 eine zweite Ansicht der erfindungsgemäßen Vorrichtung gemäß Fig. 7;
Fig. 9 eine dritte Ansicht der erfindungsgemäßen Vorrichtung gemäß Fig. 7.

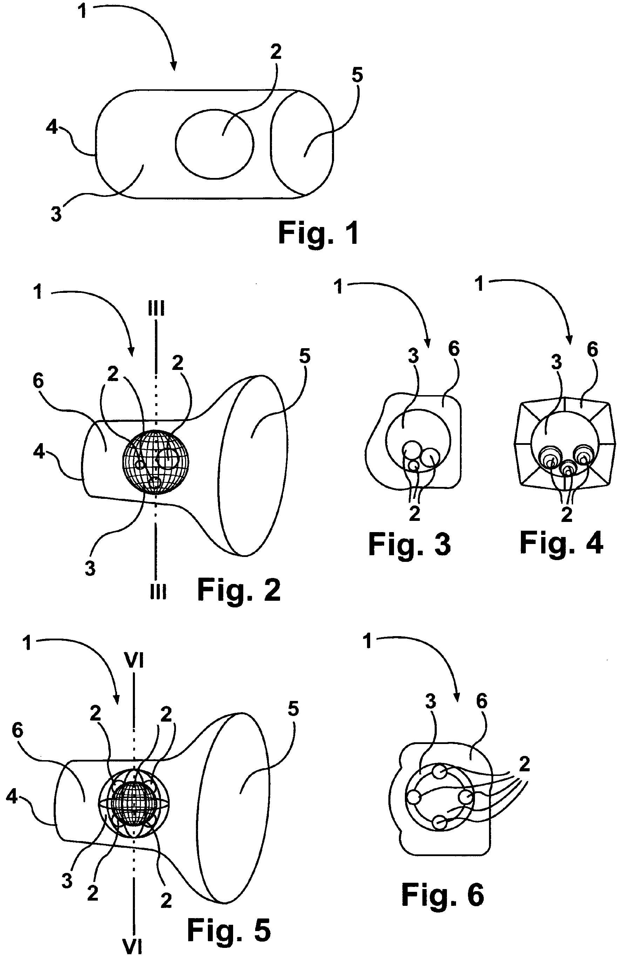

In Fig. 1 ist eine Variante einer Vorrichtung 1 dargestellt wie diese aus dem Stand der Technik bekannt ist. Die Vorrichtung 1 ist in der Ohrmuschel bzw. im Gehörgang eines Tinnituspatienten tragbar und umfasst zwei Körper 2, 3, die relativ zueinander beweglich gelagert sind. Dabei ist ein zweiter Körper 3 als Hohlkörper ausgebildet. Ein erster Körper 2 ist frei beweglich im zweiten Körper 3 gelagert. Wenn der erste Körper 2 in Bewegung versetzt wird und somit mit dem zweiten Körper 3 zusammenstößt, erzeugt die Vorrichtung 1 ein Geräusch, welches das Tinnitusgeräusch überdeckt. Der erste Körper 2 ist hier als etwa runder Körper ausgebildet. Der zweite Körper 3 ist als etwa rechteckiger Körper mit abgerundeten Kanten dargestellt, welcher ohne Probleme in den Gehörgang des Patienten einführbar ist.

Gemäß einer erfindungsgemäßen Weiterentwicklung kann vorgesehen sein, dass an einem zweiten Ende 5 der Vorrichtung 1 ein an der Ohrmuschel zur Anlage kommender Haken (nicht dargestellt) ausgebildet ist, um zu verhindern, dass die Vorrichtung 1 zu weit in den Gehörgang eingeführt wird. Die Materialwahl der Vorrichtung 1 ist dabei völlig frei und dem Fachmann überlassen. Auch die Materialwahl des zweiten Körpers 3 ist dem Fachmann überlassen. Insbesondere ist die Vorrichtung 1 aus einem biokompatiblen Material Metall wie Titan oder einer Titanlegierung oder aus einem Kunststoff gebildet. Die Vorrichtung 1 ist an einem ersten Ende 4 mit zumindest einer Öffnung ausgebildet, um den Gehörgang des die Vorrichtung 1 tragenden Patienten nicht zu verschließen. Es ist bekannt, dass es für Tinnituspatienten sehr wichtig ist, dass der Gehörgang offen ist, um das Tinnitusgeräusch nicht zu verstärken. In einer Weiterentwicklung der Fig. 1 können Öffnungen am ersten Ende 4 der Vorrichtung 1 beispielsweise als Gitter ausgebildet sein, um zu gewährleisten, dass der erste Körper 2 im zweiten Körper 3 eingeschlossen bleibt. Weiter weist die Vorrichtung 1 auch am zweiten Ende 5 zumindest eine Öffnung auf. Durch diese Öffnung ist zum einen ein permanenter Luftaustausch zwischen Gehörgang und Umwelt sichergestellt und zum anderen sind alle sonstigen Geräusche von außen für den Patienten hörbar, wenn dieser die Vorrichtung 1 in der Ohrmuschel trägt. Die Vorrichtung 1 ist in die Ohrmuschel bzw. den Gehörgang eines Patienten, welcher an Tinnitus leidet, einführbar und durch Bewegungen des Kopfes des Patienten sind die zueinander beweglich gelagerten Körper 2, 3 in Bewegung versetzbar. Dabei wird ein Geräusch erzeugt, welches größer ist als das Tinnitusgeräusch und somit für den Patienten beruhigend wirkt. Im Gegensatz zu Vorrichtungen zur Behandlung von Tinnitus, welche aus dem Stand der Technik bekannt sind, kommt die gesamte Vorrichtung 1 ohne jegliche Elektrik oder Elektronik aus. Der gesamte Mechanismus der Vorrichtung 1 und allen Varianten der Vorrichtung 1 beruht auf mechanischen Vorgängen.

Fig. 2 zeigt eine Variante der erfindungsgemäßen Vorrichtung 1. Bei dieser Ausführungsform sind die zueinander beweglich gelagerten Körper 2, 3 von einer Hülle 6 umgeben. Die Hülle 6 ist dabei etwa der Form der Ohrmuschel als Negativ dazu angeglichen und ermöglicht somit einen hohen Tragekomfort für den Patienten. Die Vorrichtung 1 weist wiederum an beiden Enden 4, 5 zumindest eine Öffnung auf, um einen Luftfluss von außen zum Gehörgang zu ermöglichen. Bei dieser Variante der erfindungsgemäßen Vorrichtung 1 sind drei etwa kugelförmige erste Körper 2 in einem als Hohlkörper ausgeführten, etwa kugelförmigen zweiten Körper 3 frei beweglich gelagert angeordnet.

Fig. 3 zeigt eine Variante der Vorrichtung 1 gemäß Fig. 2 im Querschnitt entlang der Linie III-III in Fig. 2. Die drei ersten Körper 2 sind so ausgebildet, dass der Schwerpunkt jedes einzelnen ersten Körpers 2 im Mittelpunkt des jeweils ersten Körpers 2 liegt, wobei die ersten Körper 2 als volle Körper, hohle Körper oder mit einer variierenden Dichte ausgebildet sein können. Durch diese Maßnahme sind die ersten Körper 2 drehsymmetrisch bezüglich jeder Achse durch den Mittelpunkt und erzeugen somit durch Aneinanderstoßen untereinander bzw. mit dem zweiten Körper 3 ein relativ gleichmäßiges Geräusch. Es ist nicht zwingend, kann jedoch vorgesehen sein, dass wie in Fig. 2 bzw. 3 dargestellt die drei ersten Körper 2 unterschiedlich groß sind. Durch ein Variieren der Größe der ersten Körper 2 ist das durch Aneinanderstoßen erzeugte Geräusch veränderbar und somit an die Individualbedürfnisse des Patienten anpassbar. Der zweite Körper 3 ist in Fig. 3 als Hohlkörper ausgebildet. Es kann vorgesehen sein, dass die Hülle 6 dem zweiten Körper 3 entspricht, wobei die Dichteverteilung der die ersten Körper 2 umfassenden Hülle 6 größtenteils gleichmäßig ist.

In Fig. 4 ist eine weitere Variante der Vorrichtung 1 gemäß Fig. 2 in einem Querschnitt analog der Linie III-III in Fig. 2 dargestellt. Im Gegensatz zu Fig. 3 weisen die ersten Körper 2 je einen Schwerpunkt auf, welcher sich nicht im Mittelpunkt des je ersten Körpers 2 befindet. Weiter ist die Hülle 6 als dreidimensionales Gitter ausgebildet. Dabei umfasst die Hülle 6 entweder den zweiten Körper 3 oder es handelt sich bei der Hülle 6 selbst um den zweiten Körper 3. Durch das Gitter ist wiederum die Art des beim Aneinanderstoßen der ersten Körper 2 mit dem zumindest einen zweiten Körper 3 und somit die Lautstärke des erzeugten Geräusches definiert.

Fig. 5 zeigt eine weitere Variante der erfindungsgemäßen Vorrichtung 1. Diese Variante umfasst fünf beweglich gelagerte erste Körper 2, wobei vier erste Körper 2 jeweils dieselben Maße haben und einen größeren ersten Körper 2 umringen. Dabei sind alle fünf ersten Körper 2 beweglich zueinander gelagert. Der zweite Körper 3 ist wie in Fig. 2 entweder gleich der Hülle 6 oder ist umringt von der Hülle 6 gelagert. Die Vorrichtung 1 ist wiederum der Ohrform bzw. Ohrmuschel angepasst und weist auf jeder Seite 4, 5 zumindest eine Öffnung auf. Fig. 6 zeigt eine Vorrichtung 1 gemäß Fig. 5 in einem Querschnitt entlang der Linie VI-VI in Fig. 5. Die Lagerung der Körper 2, 3 ist nochmals hervorgehoben, wobei die Dichte der einzelnen Körper 2, 3 verschieden oder jeweils gleich groß sein kann.

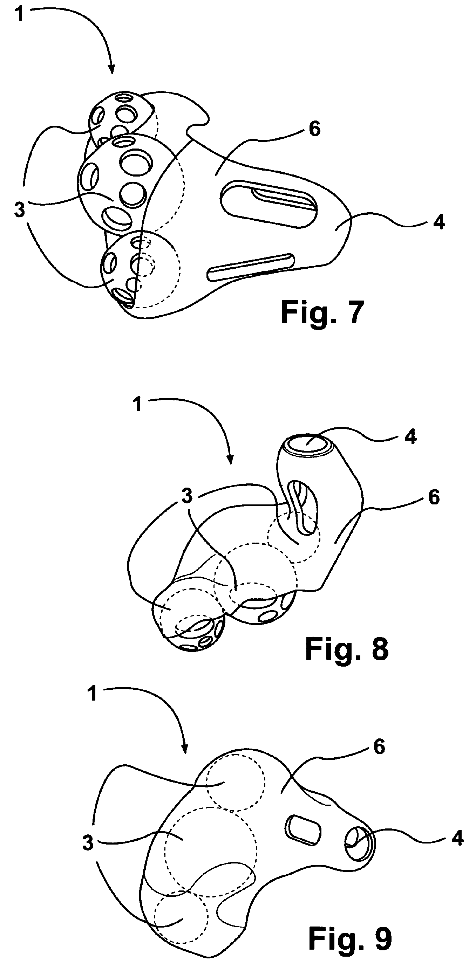

Fig. 7 bis 9 zeigen eine weitere Variante der erfindungsgemäßen Vorrichtung 1 aus verschiedenen Ansichten. Diese Variante umfasst mehrere zweite Körper 3, wobei drei zweite Körper 3 dargestellt sind. Die Lage der zweiten Körper 3 in der Vorrichtung 1 kann beliebig gewählt sein. In jedem der zweiten Körper 3 sind ein oder mehrere erste Körper 2 frei beweglich gelagert eingeschlossen. Die ersten Körper 2 sind zur besseren Übersicht nicht dargestellt und die Dichteverteilungen, Formen und Größen der ersten Körper 2 können entsprechend den Ausführungen zu Fig. 2 beliebig gewählt sein, je nachdem, welche Eigenschaften die erzeugten Geräusche haben sollen. Weiter weisen die zweiten Körper 3 zumindest eine Öffnung auf, welche kleiner ist der zumindest eine erste Körper 2. Die zweiten Körper 3 sind in einer Hülle 6 gelagert, welche mit einem ersten Ende 4, welches zumindest eine Öffnung aufweist, in den Gehörgang des Tinnituspatienten einführbar ist. Bei einer Vorrichtung 1 gemäß den Fig. 7 bis 9 handelt es sich um eine sogenannte Otoplastik. Dabei wird ein Abdruck der Ohrmuschel bzw. eines Teils des Gehörganges des Patienten genommen, anschließend davon ein digitales dreidimensionales Model erstellt und schließlich anhand dieses Models die Otoplastik hergestellt. Die so produzierte Vorrichtung 1 passt perfekt in die Ohrmuschel des Patienten und ist somit sehr angenehm zu tragen. Bei Otoplastiken können zwei Varianten unterschieden werden. Zum einen eine Otoplastik, welche für jeden Patienten individuell hergestellt ist, und zum anderen eine Otoplastik, welche für ein "Norm-Ohr", also ein durchschnittliches Ohr hergestellt ist.

Alle Varianten der Vorrichtung 1 werden vorzugsweise mit einem 3D-Drucker hergestellt. Bevorzugt wird die gesamte Vorrichtung 1 einteilig in einem Arbeitsschritt hergestellt, es kann jedoch auch vorgesehen sein, dass die Vorrichtung 1 aus mehreren Teilen besteht, wobei zumindest ein Teil mit einem 3D-Drucker hergestellt wird. In den Fig. 7 bis 9 kann z. B. vorgesehen sein, dass die Hülle 6 und die zweiten Körper 3 mitsamt den ersten Körpern 2 in zwei getrennten Verfahren hergestellt werden und anschließend miteinander verbunden werden, insbesondere durch Kleben. Dieses Verfahren hat den Vorteil, dass dabei für die Hülle 6 und die Körper 2, 3 verschiedene Materialien verwendet werden können. Die Vorteile der Herstellung der Vorrichtung 1 mit einem 3D-Drucker sind darin zu sehen, dass diese Methode so gut wie keinen Materialabfall produziert und zur Herstellung keine Formen oder dergleichen notwendig sind. Insbesondere ist die Methode sehr gut geeignet zum Herstellen von einem ersten Körper 2 in einem zweiten Körper 3, da diese Ausführungsform ohne Zusammenkleben des zweiten Körpers 3 nach Einführen des ersten Körpers 2 auskommt. Beide Körper 2, 3 mitsamt der zueinander beweglichen Lagerung können in nur einem Arbeitsschritt in relativ einfacher Art und Weise hergestellt werden. Als Material für die Vorrichtung 1 eignet sich jedes erdenkliche Material, insbesondere ein Material, welches von einem 3D-verarbeitet werden kann und biokompatibel ist, z. B. Titan oder eine Titanlegierung oder ein Kunststoff. Besteht die Vorrichtung 1 aus mehreren Einzelteilen, kommt für die Teile, welche nicht mit einem 3D-Drucker hergestellt werden, jedes erdenkliche Material infrage. Beispielsweise kann die Hülle 6 aus einem Kunststoff bestehen, wohingegen die Körper 2, 3 aus Titan bzw. einer Titanlegierung gebildet sein können.

## Patentansprüche

1. In der Ohrmuschel tragbare Vorrichtung (1) zum Überdecken eines Tinnitusgeräusches, wobei zumindest zwei Körper (2, 3) vorgesehen sind, welche relativ zueinander beweglich gelagert sind, um durch Aneinanderstoßen ein das Tinnitusgeräusch überdeckendes Geräusch zu erzeugen, **dadurch gekennzeichnet, dass** die zumindest zwei Körper (2, 3) als kugelförmige Körper ausgebildet sind.

2. Vorrichtung (1) nach Anspruch 1, **dadurch gekennzeichnet, dass** an einem ersten Ende (4), welches in den Gehörgang einführbar ist, zumindest eine Öffnung ausgebildet ist.

3. Vorrichtung (1) nach Anspruch 1 oder 2, **dadurch gekennzeichnet, dass** ein zweites Ende (5), welches die Vorrichtung (1) nach außen hin abschließt, zumindest eine Öffnung aufweist.

4. Vorrichtung (1) nach einem der Ansprüche 1 bis 3, **dadurch gekennzeichnet, dass** zur Erzeugung des Geräusches zumindest ein erster Körper (2) frei beweglich in einem zweiten Körper (3) gelagert ist, wobei der zweite Körper (3) als Hohlkörper ausgebildet ist.

5. Vorrichtung (1) nach Anspruch 4, **dadurch gekennzeichnet, dass** der zumindest eine zweite Körper (3) zumindest eine Öffnung aufweist, wobei die zumindest eine Öffnung kleiner als der zumindest eine erste Körper (2) ist.

6. Vorrichtung (1) nach einem der Ansprüche 1 bis 5, **dadurch gekennzeichnet, dass** die zueinander gerichteten Oberflächen der zumindest zwei Körper (2, 3) als raue Oberflächen ausgebildet sind.

7. Vorrichtung (1) nach einem der Ansprüche 1 bis 6, **dadurch gekennzeichnet, dass** die zumindest zwei Körper (2, 3) relativ zueinander in Bewegung versetzbar sind, wobei die Bewegung frei von elektrischen Mitteln erzeugbar ist.

8. Vorrichtung (1) nach einem der Ansprüche 1 bis 7, **dadurch gekennzeichnet, dass** keine elektrischen Bauelemente vorhanden sind.

9. Vorrichtung (1) nach einem der Ansprüche 1 bis 8, **dadurch gekennzeichnet, dass** die Vorrichtung (1) der Ohrform passgenau anpassbar ist.

10. Vorrichtung (1) nach einem der Ansprüche 1 bis 9, **dadurch gekennzeichnet, dass** die Vorrichtung (1) mit einem 3D-Drucker hergestellt ist.

11. Verfahren zum Herstellen einer Vorrichtung (1) zum Überdecken eines Tinnitusgeräusches, insbesondere durch eine in der Ohrmuschel tragbare Vorrichtung (1) nach einem der Ansprüche 1 bis 10, wobei die Vorrichtung (1) zumindest teilweise mit einem 3D-Drucker hergestellt wird wobei zumindest zwei Körper (2, 3) relativ zueinander beweglich gelagert werden, um durch Aneinanderstoßen ein das Tinnitusgeräusch überdeckendes Geräusch zu erzeugen, und wobei die zumindest zwei Körper (2,3) als kugelförmige Körper ausgebildet sind.

12. Verfahren nach Anspruch 11, **dadurch gekennzeichnet, dass** ein erster Körper (2) frei beweglich in einem zweiten Körper (3) gelagert wird, wobei der zweite Körper (3) als Hohlkörper ausgebildet wird.

13. Verfahren nach Anspruch 11 oder 12, **dadurch gekennzeichnet, dass** die Vorrichtung (1) einteilig hergestellt wird.

14. Verfahren nach einem der Ansprüche 11 bis 13, **dadurch gekennzeichnet, dass** ein Abdruck einer menschlichen Ohrmuschel angefertigt wird, wonach ein digitales Modell der Ohrmuschel erzeugt wird und in weiterer Folge die Vorrichtung (1) mit einem 3D-Drucker hergestellt wird.

## Claims

1. A device (1) that can be worn in the external ear for masking a tinnitus noise, wherein at least two bodies (2, 3) are provided, which are mounted mobile relative to one another, in order to generate a noise masking the tinnitus noise by striking each other, **characterised in that** the at least two bodies (2, 3) are constituted as spherical bodies.

2. The device (1) according to claim 1, **characterised in that** at least one opening is constituted at the first end (4) which can be introduced into the auditory canal.

3. The device (1) according to claim 1 or 2, **characterised in that** a second end (5), which terminates the device (1) to the exterior, comprises at least one opening.

4. The device (1) according to any one of claims 1 to 3, **characterised in that** at least a first body (2) is mounted freely mobile in a second body (3) in order to generate the noise, wherein the second body (3) is constituted as a hollow body.

5. The device (1) according to claim 4, **characterised in that** the at least one second body (3) comprises at least one opening, wherein the at least one opening is smaller than the at least one first body (2).

6. The device (1) according to any one of claims 1 to 5, **characterised in that** the mutually facing surfaces of the at least two bodies (2, 3) are constituted as rough surfaces.

7. The device (1) according to any one of claims 1 to 6, **characterised in that** the at least two bodies (2, 3) can be set into motion relative to one another, wherein the motion can be generated free from electrical means.

8. The device (1) according to any one of claims 1 to 7, **characterised in that** no electrical components are present.

9. The device (1) according to any one of claims 1 to 8, **characterised in that** the device (1) is adapted with a precise fit to the shape of the ear.

10. The device (1) according to any one of claims 1 to 9, **characterised in that** the device (1) is produced with a 3D printer.

11. A method for producing a device (1) for masking a tinnitus noise, in particular by means of a device (1) that can be worn in the external ear according to any one of claims 1 to 10, wherein the device (1) is produced at least partially with a 3D printer, wherein at least two bodies (2, 3) are mounted mobile relative to one another, in order to generate a noise masking the tinnitus noise by striking each other, and wherein the at least two bodies (2, 3) are constituted as spherical bodies.

12. The method according to claim 11, **characterised in that** the first body (2) is mounted freely mobile in a second body (3), wherein the second body (3) is constituted as a hollow body.

13. The method according to claim 11 or 12, **characterised in that** the device (1) is produced in one piece.

14. The method according to any one of claims 11 to 13, **characterised in that** a cast of the human external ear is prepared, after which a digital model of the external ear is produced and the device (1) is then produced with a 3D printer.

## Revendications

1. Dispositif (1) susceptible d'être porté dans le pavillon de l'oreille, pour couvrir les acouphènes, au moins deux corps (2, 3) étant prévus qui sont logés en étant mobiles l'un par rapport à l'autre, pour générer par collision un bruit couvrant les acouphènes, **caractérisé en ce que** les au moins deux corps (2, 3) sont conçus en tant que corps sphériques.

2. Dispositif (1) selon la revendication 1, **caractérisé en ce que** sur une première extrémité (4), laquelle est insérable dans le conduit auditif est conçu au moins un orifice.

3. Dispositif (1) selon la revendication 1 ou la revendication 2, **caractérisé en ce qu'**une deuxième extrémité (5) qui referme le dispositif (1) sur l'extérieur comporte au moins un orifice.

4. Dispositif (1) selon l'une quelconque des revendications 1 à 3, **caractérisé en ce que** pour générer le bruit, au moins un premier corps (2) est logé en étant librement mobile dans un deuxième corps (3), le deuxième corps (3) étant conçu en tant que corps creux.

5. Dispositif (1) selon la revendication 4, **caractérisé en ce que** l'au moins un deuxième corps (3) comporte au moins un orifice, l'au moins un orifice étant plus petit que l'au moins un premier corps (2).

6. Dispositif (1) selon l'une quelconque des revendications 1 à 5, **caractérisé en ce que** les surfaces dirigées l'une vers l'autre des au moins deux corps (2, 3) sont conçues en tant que surfaces rugueuses.

7. Dispositif (1) selon l'une quelconque des revendications 1 à 6, **caractérisé en ce que** les au moins deux corps (2, 3) sont susceptibles d'être amenés en déplacement l'un par rapport à l'autre, le déplacement pouvant être généré sans aucun moyen électrique.

8. Dispositif (1) selon l'une quelconque des revendications 1 à 7, **caractérisé en ce qu'**aucun composant électrique n'est présent.

9. Dispositif (1) selon l'une quelconque des revendications 1 à 8, **caractérisé en ce que** le dispositif (1) est exactement ajustable à la forme de l'oreille.

10. Dispositif (1) selon l'une quelconque des revendications 1 à 9, **caractérisé en ce que** le dispositif (1) est fabriqué avec une imprimante en 3D.

11. Procédé destiné à fabriquer un dispositif (1) pour couvrir des acouphènes, notamment par un dispositif (1) qui peut se porter dans le pavillon de l'oreille selon l'une quelconque des revendications 1 à 10, le dispositif (1) étant fabriqué au moins en partie avec une imprimante en 3D, aux moins deux corps (2, 3) étant logés en étant mobiles l'un vers l'autre, pour générer par collision un bruit couvrant les acouphènes et les au moins deux corps (2, 3) étant conçus en tant que corps sphériques.

12. Procédé selon la revendication 11, **caractérisé en ce qu'**un premier corps (2) est logé en étant librement mobile dans un deuxième corps (3), le deuxième corps (3) étant conçu en tant que corps creux.

13. Procédé selon la revendication 11 ou la revendication 12, **caractérisé en ce que** le dispositif (1) est fabriqué en monobloc.

14. Procédé selon l'une quelconque des revendications 11 à 13, **caractérisé en ce qu'**on réalisé une empreinte d'une oreille humaine, suite à quoi, on génère un modèle numérique du pavillon de l'oreille et par la suite, on fabrique le dispositif (1) avec une imprimante en 3D.
